# EUROPÄISCHE PATENTSCHRIFT

(11) **EP 3 215 597 B1**
(45) Veröffentlichungstag und Bekanntmachung des Hinweises auf die Patenterteilung: **18.03.2020**
(21) Anmeldenummer: 15784364.0
(22) Anmeldetag: 22.10.2015
(51) Int. Cl.: C12M 1/107, C12M 3/00, C12M 1/06, C12M 1/00, C12M 1/02

(54) **TEMPERIERVORRICHTUNG**
TEMPERATURE CONTROL APPARATUS
DISPOSITIF DE MISE EN TEMPÉRATURE

(30) Priorität: 06.11.2014 DE 102014016297
(43) Veröffentlichungstag der Anmeldung: 13.09.2017
(73) Patentinhaber: Xylem Europe GmbH, 8200 Schaffhausen (CH)
(72) Erfinder: STILLER, Wilfried, 25488 Holm (DE); LÜDERSEN, Ulrich, 21423 Winsen / Luhe (DE); BALSSEN, Eilert, 30900 Wedemark (DE)
(74) Vertreter: Lenzing Gerber Stute
(86) Internationale Anmeldenummer: PCT/EP2015/074493
(87) Internationale Veröffentlichungsnummer: WO 2016/071118

(56) Entgegenhaltungen:
- EP-A1- 0 025 571
- EP-A2- 1 130 336
- EP-A2- 2 602 020
- EP-A2- 2 628 958
- WO-A1-2014/033075
- WO-A2-2009/040330
- CA-A- 1 102 019
- DE-A1- 2 821 790
- DE-A1-102009 002 925
- DE-A1-102009 041 569

## Beschreibung

Die vorliegende Erfindung betrifft eine Temperiervorrichtung mit den Merkmalen des Oberbegriffs des Anspruchs 1 und eine Biogasanlage mit den Merkmalen des Oberbegriffs des Anspruchs 8.

In Biogasanlagen sind Bakterien für den Abbau und die Umsetzung von Biomasse zu Biogas verantwortlich. Dabei finden die Bakterien nur bei einer bestimmten Temperatur optimale Lebensbedingungen vor, bei der der biochemische Prozess effektiv abläuft. Zur mikrobiellen Umsetzung der Biomasse zu Biogas ist es daher notwendig, die Temperatur im Faulbehälter konstant zu halten. Im mesophilen Betrieb liegt die Temperatur dabei idealerweise bei circa 35°C bis 40°C. Zudem ist zur Versorgung der Bakterien mit frischen Nährstoffen eine Umwälzung der im Faulbehälter enthaltenden Biomasse notwendig.

Herkömmlicherweise werden aus Rohren angefertigte zylindrische oder flache Heizkörper mit Warmwasserdurchfluss als Wärmetauscher zur Erwärmung der Biomasse in Faulbehältern eingesetzt. Dabei sind beispielsweise aus der deutschen Offenlegungsschrift 28 21 790 und der JP 3 161039 A Heizkörper bekannt, die in Zusammenwirkung mit Rührern die Biomasse erwärmen, damit die Biomasse möglichst gleichmäßig und effektiv temperiert wird.

DE 10 2009 002 925 A1 offenbart ein Heiz- und Rührregister, bei dem die Heizregister so zu einem Rührer positioniert sind, dass eine von dem Rührer erzeugte Strömung die Heizregister zumindest teilweise durchströmen und umströmen kann. Die Heizregister und der Rührer bilden dabei eine Einheit, die gemeinsam in den Faulbehälter eingesetzt werden kann. Ein Vorteil dabei ist, dass bei dem Einbau der kombinierten Baugruppe nicht auf den geeigneten Abstand zwischen Rührer und Heizregister geachtete werden muss und der Einbau in einen gefüllten Faulbehälter möglich ist. Das Heizregister ist aus Rohren gebildet, die in einer Ebene quer zur Strömungsrichtung der Biomasse angeordnet sind, um die Wärmeübertragungsfläche zu vergrößern. Ein Nachteil der hier verwendeten Heizregister ist, dass die von der Biomasse durchströmten Zwischenräume einen begrenzten freien Querschnitt aufweisen, an denen sich unerwünschte Ablagerungen oder zeitweilige Verstopfungen bilden können.

CA1102019 offenbart einen anaeroben Fermenter mit einer kombinierten Misch- und Heizeinrichtung, bei der mittels Impeller der Massenstrom durch ein von einem doppelwandigen Rohr gebildeten Innenvolumen strömt.

Der Erfindung liegt die Aufgabe zugrunde, eine Temperiervorrichtung anzugeben, die möglichst gleichmäßig und effektiv Biomasse temperiert und gleichzeitig so gestaltet ist, dass Ablagerungen der Biomasse an ihr vermieden werden können. Zudem ist eine Biogasanlage anzugeben, die eine Temperiervorrichtung mit den gewünschten Eigenschaften aufweist.

Diese Aufgabe wird von einer Temperiervorrichtung mit den Merkmalen des Anspruchs 1 und einer Biogasanlage mit den Merkmalen des Anspruchs 8 gelöst.

Danach ist eine Temperiervorrichtung zum Temperieren von in einem Behälter enthaltender Biomasse vorgesehen, aufweisend wenigstens ein Mittel zur Temperierung und wenigstens eine Fördereinrichtung zum Befördern eines Massenstroms von Biomasse entlang einer Hauptstromrichtung, wobei die wenigstens eine Fördereinrichtung wenigstens einen Rührer aufweist, und wobei das wenigstens eine Mittel zur Temperierung in der von der wenigstens einen Fördereinrichtung erzeugten Hauptstromrichtung angeordnet ist, wobei das wenigstens eine Mittel zur Temperierung rohrförmig mit einem Innenrohr und mit einem zu dem Innenrohr koaxial angeordnetem Außenrohr ausgebildet ist, und wobei das Innenrohr und das Außenrohr einen Zwischenraum bilden, der von einem Temperiermedium durchströmbar ist, und wobei das wenigstens eine Mittel zur Temperierung so zu der wenigstens einen Fördereinrichtung orientiert ist, dass in einem Betrieb der Temperiervorrichtung vorzugsweise wenigstens 40% des Fördervolumens des von der wenigstens einen Fördereinrichtung erzeugten Massenstroms durch das Innenrohr strömt. Der Rührer bewegt die Biomasse durch den rohrförmigen Wärmetauscher. Der rohrförmige Aufbau ist vorteilhaft, da die Oberfläche im wesentlichen parallel zur Strömungsrichtung der Biomasse orientiert ist und somit Ablagerungen und Verstopfungen verhindert werden können. Weiterhin bietet der rohrförmige Aufbau eine große Kontaktfläche, über die die Temperierung der Biomasse erfolgt. Durch die vom Rührer erzeugte gezielte Strömung wird der Durchsatz des Wärmetauschers an Biomasse erhöht, was zu einer gleichmäßigen Temperaturverteilung der Biomasse im Behälter führt. Weiter ist das wenigstens eine Mittel zur Temperierung stromabwärts der wenigstens einen Fördereinrichtung angeordnet und so orientiert, dass die Längsachse des wenigstens einen Mittels zur Temperierung mit einer Rotationsachse des wenigstens einen Rührers übereinstimmt. Außerdem ist die Fördereinrichtung so dimensioniert, dass ein Durchmesser einer Einhüllenden der wenigstens einen Fördereinrichtung in einem Bereich zwischen 100-140% des Innendurchmessers des Innenrohrs des wenigstens einen Mittel zur Temperierung liegt. Dadurch kann garantiert werden, dass ein Teil des Massenstroms durch das Innenrohr und der andere Teil entlang der Außenseite des wenigstens eine Mittels zur Temperierung strömt. Vorzugsweise ist vorgesehen, dass mehr als 75% des Fördervolumens des von der wenigstens einen Fördereinrichtung erzeugten Massenstroms durch das Innenrohr strömt. Dabei ist es vorteilhaft, wenn sogar mehr als 90% des Fördervolumens des von der wenigstens einen Fördereinrichtung erzeugten Massenstroms durch das Innenrohr strömt.

Das Mittel zur Temperierung ist ein Wärmetauscher.

In einer Ausführungsform ist vorgesehen, dass die wenigstens eine Fördereinrichtung ein einziger Rührer ist.

Damit die Temperiervorrichtung möglichst kompakt ist, weist sie eine einen Fuß aufweisende Haltevorrichtung auf, wobei der Fuß zum Aufstellen der Temperiervorrichtung auf einen Boden eines Behälters ausgebildet ist, und wobei an der Haltevorrichtung die wenigstens eine Fördereinrichtung und das wenigstens eine Mittel zur Temperierung gehalten sind. Die Haltevorrichtung kann fest in dem Behälter installiert sein, sie kann aber auch portable ausgestaltet sein.

Es ist bevorzugt vorgesehen, dass die von der wenigstens einen Fördereinrichtung erzeugte Hauptstromrichtung parallel zum Fuß orientiert ist.

Weiterhin ist eine Biogasanlage mit einem Behälter zur Aufnahme der Biomasse und mit einer Temperiervorrichtung zum Temperieren der in dem Behälter enthaltenden Biomasse mit wenigstens einer der vorhergehend genannten Eigenschaften vorgesehen.

Dabei ist es vorteilhaft, wenn der Behälter kreiszylindrisch ist und die Temperiervorrichtung in dem Behälter so angeordnet ist, dass die Hauptstromrichtung in Richtung der Mitte des Behälters und unter einem Winkel von 10 bis 40 Grad gegen einen Radius des Bodens orientiert ist. Bei größeren Behältern ist ein kleinerer Winkel bevorzugt, damit eine gute Durchmischung und Temperierung gewährleistet werden kann. Zur gleichmäßigen Durchmischung der Biomasse, können vorzugsweise wenigstens zwei weitere Rührer in dem Behälter vorgesehen sind, die an einer Wandung des Behälters angeordnet sind und mit der Heizvorrichtung gleichmäßig über den Behälter verteilt sind.

Nachfolgend wird ein Ausführungsbeispiel der Erfindung anhand der Zeichnungen näher beschrieben. Es zeigen:
- Fig. 1:: eine räumliche Ansicht einer Temperiervorrichtung mit einem Rührer,
- Fig. 2:: eine Seitenansicht einer Temperiervorrichtung mit einem Rührer,
- Fig. 3:: eine Ansicht von oben einer Biogasanlage mit Behälter und einer darin angeordneten Temperiervorrichtung gemäß Fig. 1, sowie
- Fig. 4:: eine räumliche Ansicht einer Biogasanlage mit Behälter und einer darin angeordneten Temperiervorrichtung gemäß Fig. 1.

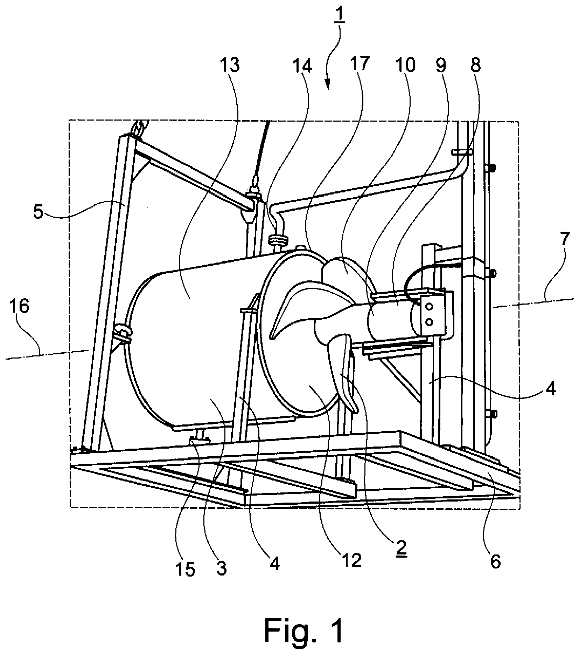

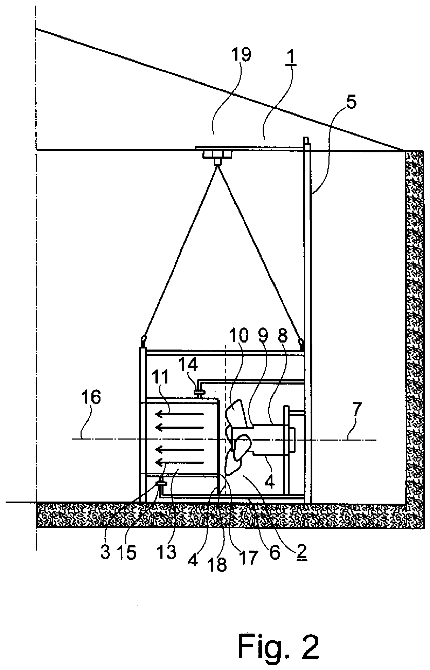

In den Figuren 1 und 2 ist eine Temperiervorrichtung 1 für Behälter von Biogasanlagen mit einem Rührer 2 und einem Wärmetauscher 3 gezeigt. Der Wärmetauscher 3 und der Rührer 2 sind über Träger 4 mit einer Haltevorrichtung 5 verbunden. Die Haltevorrichtung 5 weist einen Bodenrahmen 6 (Fuß) zum Aufstellen der Temperiervorrichtung 1 auf den Boden eines Behälters auf. Der Rührer 2 ist mit seiner Drehachse 7 parallel zum Bodenrahmen 6 angeordnet. Der Rührer 2 ist mit einem Antrieb 8 direkt verbunden. Der Antrieb 8 befindet sich somit im Betrieb der Temperiervorrichtung im Inneren des Behälters bzw. der Biomasse. Der Rührer 2 weist drei auf einer Rührwelle 9 montierte Rührerblätter 10 auf, die von dem Antrieb 8 angetrieben um die Drehachse 7 rotieren. Dabei sind die Rührerblätter 10 so ausgestaltet, dass auf der antriebsnahen Seite die Biomasse angesogen wird und auf der antriebsfernen Seite die Biomasse von dem Rührer 2 weggeschoben wird. Dadurch ergibt sich eine überwiegend axiale vom Antrieb 8 weg gerichtete Hauptströmung 11 der Biomasse. Stromabwärts des Rührers 2, auf der antriebsfernen Seite ist der Wärmetauscher 3 angeordnet. Der Wärmetauscher 3 ist rohrförmig und doppelwandig ausgestaltet. Der Wärmetauscher 3 weist ein von der Biomasse durchströmbares Innenrohr 12 auf und ein dazu zu einer Längsachse koaxial angeordnetes Außenrohr 13. Der zwischen Innenrohr 12 und Außenrohr 13 gebildete Zwischenraum wird im Betrieb von einem Temperiermedium durchströmt. Der Zwischenraum ist in einem rührernahen Bereich mit einem Temperiermediumablauf 14 und in einem rührerfernen Bereich mit einem Temperiermediumzulauf 15 verbunden. Der Ablauf 14 und der Zulauf 15 sind dabei diametral angeordnet. Der Wärmetauscher 3 ist so positioniert, dass seine Längsachse 16 mit der Drehachse 7 des Rührers 2 übereinstimmt. Der Innendurchmesser des Wärmetauschers 3 ist etwas kleiner als der Durchmesser der Einhüllenden des Rührers 2 bzw. der Rührerblätter 10. Der Wärmetauscher 3 ist vorzugsweise zwischen 1 m und 3 m lang. Der Wärmetauscher 3 ist direkt in Strömungsrichtung 11 hinter dem Rührer 2 angeordnet. Der Abstand zwischen einer rührernahen Stirnseite 17 des Wärmetauschers 3 und einem durch die Rührerblätter 10 gebildetem äußerem Ende 18 des Rührers 2 liegt in einem Bereich zwischen 10 cm bis 3 m.

Die Haltevorrichtung 5 weist weiterhin eine Transportvorrichtung 19 auf, die im Betrieb aus der im Behälter vorhandenen Biomasse herausragt. Mittels der Transportvorrichtung 19 kann die Temperiervorrichtung 1 als Wärmetauscher 3 und Rührer 2 aufweisendes Bauelement auch bei mit Biomasse gefülltem Behälter entnommen, versetzt oder eingesetzt werden, ohne dass eine unerwünschte Entleerung des Behälters notwendig ist.

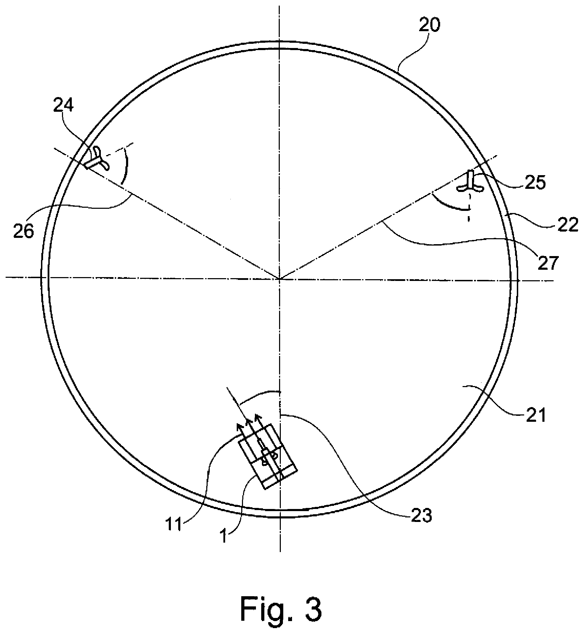

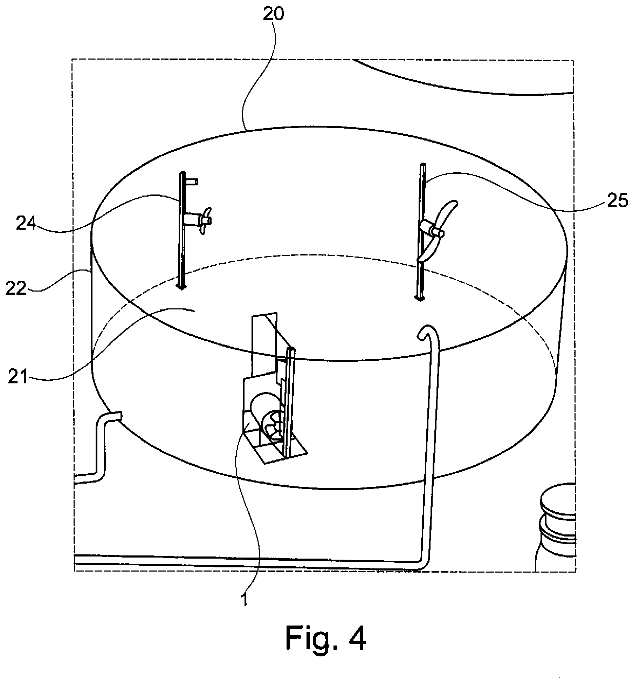

Die Temperiervorrichtung 1 ist in einem kreiszylindrischen Behälter 20 vorzugsweise, wie in Figur 3 und 4 dargestellt, angeordnet. Der Behälter 20 weist einen kreisförmigen Boden 21 und eine senkrecht dazu angeordnete Wandung 22 auf. Die Längsachse 16 des Wärmetauschers 3 bzw. die Drehachse 7 des Rührers 2 ist in einem Winkel von 30° zum Radius 23 des Bodens 21 des Behälters 20 ausgerichtet, so dass der Rührer 2 die Biomasse von der Wandung 22 weg zum Inneren des Behälters 20 hin bewegt. Dabei ist der Abstand von der Wandung 22 idealerweise in einer Größenordnung von 1 m bis 3 m zu wählen.

Um eine gute Durchmischung der Biomasse zu erreichen, sind zwei weitere Rührer 24, 25 vorgesehen. Diese beiden Rührer 24, 25 sind an der Innenseite der Wandung 22 angeordnet und unter einem Winkel von 60° zum Radius 26, 27 des Bodens 21 des Behälters 20 ausgerichtet. Die Rührer 24, 25 sind vom Mittelpunkt des Bodens ausgehend jeweils unter einem Winkel von 120° zu der Temperiervorrichtung 1 angeordnet. Dabei sind die beiden Rührer 24, 25 und die Temperiervorrichtung 1 so ausgerichtet, dass sie jeweils in Strömungsrichtung angeordnet sind und somit eine in Umfangsrichtung des kreisförmigen Bodens in etwa gleichgerichtete Hauptströmung erzeugen.

In Ansicht der Figur 3, sind die Rührer 24, 25, 2 so angeordnet, dass sich eine Strömung im Wesentlichem im Uhrzeigersinn ausbildet.

Im Betrieb wird von dem Wärmetauscher Wärme an die im Behälter enthaltende Biomasse abgegeben. Wenn bei der Umsetzung zu Biogas die Temperatur zu stark ansteigen sollte, kann durch den Wärmetauscher auch Wärme aus dem Faulbehälter abgeführt werden. Eine Kühlung der Biomasse ist somit ebenfalls möglich. Als Temperiermedium kann jeder beliebiger Wärmeträger, vorzugsweise Wasser oder Thermalöle eingesetzt werden. Der Rührer schiebt die Biomasse durch den rohrförmigen Wärmetauscher und außen vorbei. Der rohrförmige Aufbau ist vorteilhaft, da die Oberfläche im wesentlichen parallel zur Strömungsrichtung der Biomasse orientiert ist und somit Ablagerungen und Verstopfungen verhindert werden können. Weiterhin bietet der rohrförmige Aufbau eine große Kontaktfläche, über die die Temperierung der Biomasse erfolgt. Durch die vom Rührer erzeugte gezielte Strömung wird der Durchsatz des Wärmetauschers an Biomasse erhöht, was zu einer gleichmäßigen Temperaturverteilung der Biomasse im Behälter führt.

Es kann auch vorgesehen sein, mehrere Rührer hintereinander stromabwärts mit jeweils einem Wärmetauscher anzuordnen.

Die erfindungsgemäße Temperiervorrichtung kann in der Kombination fest in einen Behälter eingebaut werden. Es kann aber auch vorgesehen sein, die Temperiervorrichtung portable auszugestalten, so dass die Vorrichtung zum Beispiel zur Wartung oder zum Austausch aus dem Behälter entnommen werden kann.

Die Temperiervorrichtung kann je nach Anwendungsbereich horizontal oder vertikal in den Behälter eingebaut werden. Zum Beispiel ist bei kreiszylinderförmigen oder liegenden tankförmigen Behältern (z.B. Fermentern) eine horizontale Orientierung wünschenswert. Hingegen ist bei stehenden tankförmigen Behältern eine vertikale Orientierung der Temperiervorrichtung bevorzugt.

Der Rührer ist vorzugsweise als axial fördernder Rührer mit zwei bis vier Rührerblättern ausgeführt. Die Rührerblätter sind dabei gebogen, wodurch eine größtmögliche Geschwindigkeit der Biomasse bei langsam laufendem Rührer und so eine optimale Durchmischung erreicht wird.

Bei großen Behältern kann vorgesehen sein, dass die Temperiervorrichtung ein Rohr aufweist, dass sich an das wenigstens eine Mittel zur Temperierung in Strömungsrichtung stromabwärts anschließt. Dabei ist es bevorzugt, wenn das Rohr in etwa den gleichen Innendurchmesser wie das wenigstens eine Mittel zur Temperierung aufweist und die Fördereinrichtung bzw. der Rührer im Inneren des Wärmetauschers angeordnet ist. Diese Ausgestaltung der erfindungsgemäßen Temperiervorrichtung sorgt für eine Temperierung und Durchmischung der Biomasse mit einem großen Wirkradius. Da 100% des von dem Rührer erzeugten Massenstroms durch den Wärmetauscher und das daran angesetzte Rohr strömt und sich außerhalb des Rohres ein Rückstrom bildet, können auch entlegene Bereich des Behälters durchrührt werden.

Die erfindungsgemäße Temperiervorrichtung ist unter anderem für den Einsatz in Faulbehältern bzw. Fermentern, Hydrolysefermentern und Nachgärbehälter von Biogasanlagen sowie Behältern von Kläranlagen vorgesehen. Die Temperiervorrichtung ist so ausgestaltet, dass die Biomasse gleichmäßig und effektiv temperiert werden kann und Ablagerungen der Biomasse an der Außenseite verhindert werden.

## Patentansprüche

1. Temperiervorrichtung (1) zum Temperieren von in einem Behälter (20) enthaltener Biomasse, aufweisend
- wenigstens ein Mittel zur Temperierung (3) und
- wenigstens eine Fördereinrichtung (2) zum Befördern eines Massenstroms von Biomasse entlang einer Hauptstromrichtung,
-- wobei die wenigstens eine Fördereinrichtung (2) wenigstens einen Rührer (2) aufweist, und
-- wobei das wenigstens eine Mittel zur Temperierung (3) in der von der wenigstens einen Fördereinrichtung (2) erzeugten Hauptstromrichtung angeordnet ist, und das wenigstens eine Mittel zur Temperierung (3) rohrförmig mit einem Innenrohr (12) und mit einem zu dem Innenrohr (12) koaxial angeordnetem Außenrohr (13) ausgebildet ist,
- wobei das Innenrohr (12) und das Außenrohr (13) einen Zwischenraum bilden, der von einem Temperiermedium durchströmbar ist, und
- wobei das wenigstens eine Mittel zur Temperierung (3) so zu der wenigstens einen Fördereinrichtung (2) orientiert ist, dass wenigstens 40% des Fördervolumens des von der wenigstens einen Fördereinrichtung (2) erzeugten Massenstroms (11) durch das Innenrohr (12) strömt, **dadurch gekennzeichnet, dass**
- das wenigstens eine Mittel zur Temperierung (3) stromabwärts der wenigstens einen Fördereinrichtung (2) angeordnet ist und so orientiert ist, dass die Längsachse des wenigstens einen Mittels zur Temperierung mit einer Rotationsachse des wenigstens einen Rührers übereinstimmt, und
- dass ein Durchmesser einer Einhüllenden der wenigstens einen Fördereinrichtung (2) in einem Bereich zwischen 100-140% des Innendurchmessers des Innenrohrs (12) des wenigstens einen Mittel zur Temperierung (3) liegt.

2. Temperiervorrichtung nach Anspruch 1, **dadurch gekennzeichnet, dass** mehr als 75% des Fördervolumens des von der wenigstens einen Fördereinrichtung (2) erzeugten Massenstroms (11) durch das Innenrohr (12) strömt.

3. Temperiervorrichtung nach Anspruch 1 oder 2, **dadurch gekennzeichnet, dass** mehr als 90% des Fördervolumens des von der wenigstens einen Fördereinrichtung (2) erzeugten Massenstroms (11) durch das Innenrohr (12) strömt.

4. Temperiervorrichtung nach einem der vorhergehenden Ansprüche, **dadurch gekennzeichnet, dass** das wenigstens eine Mittel zur Temperierung (3) ein Wärmetauscher ist.

5. Temperiervorrichtung nach einem der vorhergehenden Ansprüche, **dadurch gekennzeichnet, dass** die wenigstens eine Fördereinrichtung (2) ein einziger Rührer ist.

6. Temperiervorrichtung nach einem der vorhergehenden Ansprüche, **dadurch gekennzeichnet, dass** die Temperiervorrichtung einen Fuß (6) aufweisende Haltevorrichtung (5) aufweist,
- wobei der Fuß (6) zum Aufstellen der Temperiervorrichtung (1) auf einen Boden (21) eines Behälters (20) ausgebildet ist, und
- wobei an der Haltevorrichtung (5) die wenigstens eine Fördereinrichtung (2) und das wenigstens eine Mittel zur Temperierung (3) gehalten sind.

7. Temperiervorrichtung nach Anspruch 6, **dadurch gekennzeichnet, dass** die von der wenigstens einen Fördereinrichtung (2) erzeugte Hauptstromrichtung (11) parallel zum Fuß (6) orientiert ist.

8. Biogasanlage zur Herstellung von Biogas durch mikrobielle Umsetzung von pumpfähiger Biomasse, mit einem Behälter (20) zur Aufnahme der Biomasse und mit einer Temperiervorrichtung (1) zum Temperieren der in dem Behälter (20) enthaltenden Biomasse, aufweisend
- wenigstens ein Mittel zur Temperierung (3) und
- wenigstens eine Fördereinrichtung (2) zum Befördern eines Massenstroms von Biomasse entlang einer Hauptstromrichtung,
-- wobei die wenigstens eine Fördereinrichtung (2) wenigstens einen Rührer (2) aufweist, und
-- wobei das wenigstens eine Mittel zur Temperierung (3) in der von der wenigstens einen Fördereinrichtung (2) erzeugten Hauptstromrichtung (11) angeordnet ist das wenigstens eine Mittel zur Temperierung (3) rohrförmig mit einem Innenrohr (12) und mit einem zu dem Innenrohr (12) koaxial angeordnetem Außenrohr (13) ausgebildet ist,
- wobei das Innenrohr (12) und das Außenrohr (13) einen Zwischenraum bilden, der von einem Temperiermedium durchströmbar ist, und
- wobei das wenigstens eine Mittel zur Temperierung (3) so zu der wenigstens einen Fördereinrichtung (2) orientiert ist, dass in einem Betrieb der Heizvorrichtung (1) wenigstens 40% des Fördervolumens des von der wenigstens einen Fördereinrichtung (2) erzeugten Massenstroms (11) durch das Innenrohr (12) strömt,
**dadurch gekennzeichnet, dass**
- das wenigstens eine Mittel zur Temperierung (3) stromabwärts der wenigstens einen Fördereinrichtung (2) angeordnet ist und so orientiert ist, dass die Längsachse des wenigstens einen Mittels zur Temperierung mit einer Rotationsachse des wenigstens einen Rührers übereinstimmt, und
- ein Durchmesser einer Einhüllenden der wenigstens einen Fördereinrichtung (2) in einem Bereich zwischen 100-140% des Innendurchmessers des Innenrohrs (12) des wenigstens einen Mittel zur Temperierung (3) liegt.

9. Biogasanlage nach Anspruch 8, **dadurch gekennzeichnet, dass** mehr als 75% des Fördervolumens des von der wenigstens einen Fördereinrichtung (2) erzeugten Massenstroms (11) durch das Innenrohr (12) strömt.

10. Biogasanlage nach Anspruch 8 oder 9, **dadurch gekennzeichnet, dass** der Behälter (20) kreiszylindrisch ist und die Temperiervorrichtung (1) in dem Behälter (20) so angeordnet ist, dass die Hauptstromrichtung (11) in Richtung der Mitte des Behälters (20) und unter einem Winkel von 10 bis 40 Grad gegen den Radius des Bodens (21) orientiert ist.

11. Biogasanlage nach einem der vorgehenden Ansprüche 8 bis 10, **dadurch gekennzeichnet, dass** in dem Behälter (20) wenigstens zwei weitere Rührer (24, 25) vorgesehen sind, die an einer Wandung (22) des Behälters (20) angeordnet sind und mit der Temperiervorrichtung (1) gleichmäßig über den Behälter (20) verteilt sind.

12. Biogasanlage nach einem der vorgehenden Ansprüche 8 bis 11, **dadurch gekennzeichnet, dass** die Temperiervorrichtung eine einen Fuß (6) aufweisende Haltevorrichtung (5) aufweist,
- wobei der Fuß (6) zum Aufstellen der Heizvorrichtung (1) auf einen Boden (21) des Behälters (20) ausgebildet ist, und
- wobei an der Haltevorrichtung (5) die wenigstens eine Fördereinrichtung (2) und das wenigstens eine Mittel zur Temperierung (3) gehalten sind.

## Claims

1. Temperature control apparatus (1) for controlling the temperature of biomass contained in a receptacle (20), comprising
- at least one temperature control means (3), and
- at least one conveying device (2) for conveying a mass flow of biomass along a main flow direction,
-- wherein the at least one conveying device (2) has at least one stirrer (2), and
-- wherein the at least one temperature control means (3) is arranged in the main flow direction generated by the at least one conveying device (2), and the at least one temperature control means (3) is tubular having an inner tube (12) and an outer tube (13) which is arranged coaxially with respect to the inner tube (12),
- wherein the inner tube (12) and the outer tube (13) form an intermediate space through which a temperature control medium can flow, and
- wherein the at least one temperature control means (3) is orientated in such a manner with respect to the at least one conveying device (2) that at least 40% of the conveyed volume of the mass flow (11) generated by the at least one conveying device (2) flows through the inner tube (12), **characterised in that**
- the at least one temperature control means (3) is arranged downstream of the at least one conveying device (2) and is orientated in such a manner that the longitudinal axis of the at least one temperature control means corresponds to a rotation axis of the at least one stirrer, and
- **in that** a diameter of an envelope of the at least one conveying device (2) is located in a range between 100 and 140% of the inner diameter of the inner tube (12) of the at least one temperature control means (3).

2. Temperature control apparatus according to claim 1, **characterised in that** more than 75% of the conveyed volume of the mass flow (11) produced by the at least one conveying device (2) flows through the inner tube (12).

3. Temperature control apparatus according to claim 1 or claim 2, **characterised in that** more than 90% of the conveyed volume of the mass flow (11) produced by the at least one conveying device (2) flows through the inner tube (12).

4. Temperature control apparatus according to any one of the preceding claims, **characterised in that** the at least one temperature control means (3) is a heat exchanger.

5. Temperature control apparatus according to any one of the preceding claims, **characterised in that** the at least one conveying device (2) is a single stirrer.

6. Temperature control apparatus according to any one of the preceding claims, **characterised in that** the temperature control apparatus has a retention device (5) which has a base (6),
- wherein the base (6) is constructed to erect the temperature control apparatus (1) on a bottom (21) of a receptacle (20), and
- wherein the at least one conveying device (2) and the at least one temperature control means (3) are retained on the retention device (5).

7. Temperature control apparatus according to claim 6, **characterised in that** the main flow direction (11) produced by the at least one conveying device (2) is orientated parallel with the base (6).

8. Biogas installation for producing biogas by means of microbial conversion of pumpable biomass, having a receptacle (20) for receiving the biomass and having a temperature control apparatus (1) for controlling the temperature of the biomass contained in the receptacle (20), having
- at least one temperature control means (3), and
- at least one conveying device (2) for conveying a mass flow of biomass in a main flow direction,
-- wherein the at least one conveying device (2) has at least one stirrer (2), and
-- wherein the at least one temperature control means (3) is arranged in the main flow direction (11) produced by the at least one conveying device (2), the at least one temperature control means (3) is constructed in a tubular manner with an inner tube (12) and with an outer tube (13) which is arranged coaxially with respect to the inner tube (12),
- wherein the inner tube (12) and the outer tube (13) form an intermediate space through which a temperature control medium can flow, and
- wherein the at least one temperature control means (3) is orientated in such a manner relative to the at least one conveying device (2) that during operation of the heating device (1) at least 40% of the conveyed volume of the mass flow (11) produced by the at least one conveying device (2) flows through the inner tube (12),
**characterised in that**
- the at least one temperature control means (3) is arranged downstream of the at least one conveying device (2) and is orientated in such a manner that the longitudinal axis of the at least one temperature control means corresponds to a rotation axis of the at least one stirrer, and
- a diameter of an envelope of the at least one conveying device (2) is in a range between 100 and 140% of the inner diameter of the inner tube (12) of the at least one temperature control means (3).

9. Biogas installation according to claim 8, **characterised in that** more than 75% of the conveyed volume of the mass flow (11) produced by the at least one conveying device (2) flows through the inner tube (12).

10. Biogas installation according to claim 8 or claim 9, **characterised in that** the receptacle (20) is circular-cylindrical and the temperature control apparatus (1) is arranged in the receptacle (20) in such a manner that the main flow direction (11) is orientated in the direction of the centre of the receptacle (20) and at an angle of from 10 to 40 degrees with respect to the radius of the bottom (21).

11. Biogas installation according to any one of the preceding claims 8 to 10, **characterised in that** there are at least two additional stirrers (24, 25) provided in the receptacle (20) which are arranged on a wall (22) of the receptacle (20) and which are distributed with the temperature control apparatus (1) in a uniform manner over the receptacle (20).

12. Biogas installation according to any one of the preceding claims 8 to 11, **characterised in that** the temperature control apparatus has a retention device (5) which has a base (6),
- wherein the base (6) is constructed to erect the heating device (1) on a bottom (21) of the receptacle (20), and
- wherein the at least one conveying device (2) and the at least one temperature control means (3) are retained on the retention device (5).

## Revendications

1. Dispositif de mise en température (1) pour mettre en température une biomasse contenue dans un récipient (20), présentant
- au moins un moyen pour la mise en température (3) et
- au moins un système de transport (2) pour transporter un flux massique de biomasse le long d'une direction de circulation principale,
-- le ou les systèmes de transport (2) présentant au moins un agitateur (2), et
-- le ou les moyens pour la mise en température (3) étant disposés dans la direction de circulation principale produite par le ou les systèmes de transport (2), et le ou les moyens pour la mise en température (3) prenant une forme tubulaire avec un tube interne (12) et un tube externe (13) disposé coaxialement par rapport au tube interne (12),
- le tube interne (12) et le tube externe (13) formant une chambre intermédiaire qui peut être traversée par un fluide de mise en température, et
- le ou les moyens pour la mise en température (3) étant orientés par rapport au ou aux systèmes de transport (2) de sorte qu'au moins 40 % du volume de transport du flux massique (11) produit par le ou les systèmes de transport (2) circulent à travers le tube interne (12), **caractérisé en ce que**
- le ou les moyens pour la mise en température (3) sont disposés en aval du ou des systèmes de transport (2) et sont orientés de sorte que l'axe longitudinal du ou des moyens pour la mise en température correspond à un axe de rotation du ou des agitateurs, et
- un diamètre d'une enveloppe du ou des systèmes de transport (2) se situe dans une fourchette comprise entre 100 -140 % du diamètre intérieur du tube interne (12) du ou des moyens pour la mise en température (3).

2. Dispositif de mise en température selon la revendication 1, **caractérisé en ce que** plus de 75 % du volume de transport du flux massique (11) produit par le ou les systèmes de transport (2) circule à travers le tube interne (12).

3. Dispositif de mise en température selon la revendication 1 ou 2, **caractérisé en ce que** plus de 90 % du volume de transport du flux massique (11) produit par le ou les systèmes de transport (2) circulent à travers le tube interne (12).

4. Dispositif de mise en température selon l'une des revendications précédentes, **caractérisé en ce qu'**au moins un des moyens pour la mise en température (3) est un échangeur de chaleur.

5. Dispositif de mise en température selon l'une des revendications précédentes, **caractérisé en ce qu'**au moins un système de transport (2) est un agitateur unique.

6. Dispositif de mise en température selon l'une des revendications précédentes, **caractérisé en ce que** le dispositif de mise en température présente un dispositif de maintien (5) présentant un pied (6),
- le pied (6) étant conçu pour monter le dispositif de mise en température (1) sur un fond (21) d'un récipient (20), et
- le ou les systèmes de transport (2) et le ou les moyens pour la mise en température (3) étant maintenus sur le dispositif de maintient (5).

7. Dispositif de mise en température selon la revendication 6, **caractérisé en ce que** la direction de circulation principale (11) produite par le ou les systèmes de transport (2) est orientée parallèlement au pied (6).

8. Installation de biogaz pour la production de biogaz par réalisation microbienne de biomasse pouvant être pompée, avec un récipient (20) pouvant accueillir la biomasse et avec un dispositif de mise en température (1) pour la mise en température de la biomasse contenue dans le récipient (20), présentant
- au moins un moyen pour la mise en température (3) et
- au moins un système de transport (2) pour transporter un flux massique de biomasse le long d'une direction de circulation principale,
-- le ou les systèmes de transport (2) présentant au moins un agitateur (2), et
-- le ou les moyens pour la mise en température (3) étant disposés dans la direction de circulation principale produite par le ou les systèmes de transport (2), le ou les moyens pour la mise en température (3) prenant une forme tubulaire avec un tube interne (12) et un tube externe (13) disposé coaxialement par rapport au tube interne (12),
- le tube interne (12) et le tube externe (13) formant une chambre intermédiaire qui peut être traversée par un fluide de mise en température, et
- le ou les moyens pour la mise en température (3) étant orientés par rapport au ou aux systèmes de transport (2) de sorte qu'au moins 40 % du volume de transport du flux massique (11) produit par le ou les systèmes de transport (2) circulent à travers le tube interne (12) dans un fonctionnement du dispositif de chauffage (1), **caractérisé en ce que**
- le ou les moyens pour la mise en température (3) sont disposés en aval du ou des systèmes de transport (2) et sont orientés de sorte que l'axe longitudinal du ou des moyens pour la mise en température correspond à un axe de rotation du ou des agitateurs, et
- un diamètre d'une enveloppe d'au moins un système de transport (2) se situe dans une fourchette comprise entre 100 -140 % du diamètre intérieur du tube interne (12) du ou des moyens pour la mise en température (3).

9. Installation de biogaz selon la revendication 8, **caractérisée en ce que** plus de 75 % du volume de transport du flux massique (11) produit par le ou les systèmes de transport (2) circulent à travers le tube interne (12).

10. Installation de biogaz selon la revendication 8 ou 9, **caractérisée en ce que** le récipient (20) est en forme de cylindre circulaire et le dispositif de mise en température (1) dans le récipient (20) est disposé de sorte que la direction de circulation principale (11) est orientée en direction du centre du récipient (20) et selon un angle de 10 à 40 degrés contre le rayon du fond (21).

11. Installation de biogaz selon l'une des revendications précédentes 8 à 10, **caractérisée en ce que** l'on prévoit dans le récipient (20) au moins deux autres agitateurs (24, 25) qui sont disposés sur une paroi (22) du récipient (20) et sont uniformément distribués, avec le dispositif de mise en température (1), sur le récipient (20).

12. Installation de biogaz selon l'une des revendications précédentes 8 à 11, **caractérisée en ce que** le dispositif de mise en température présente un dispositif de maintien (5) présentant un pied (6),
- le pied (6) permettant l'installation du dispositif de chauffage (1) étant conçu sur un fond (21) du récipient (20), et
- le ou les systèmes de transport (2) et le ou les moyens pour la mise en température (3) étant maintenus sur le dispositif de maintien (5).
